Europäisches Patentamt

European Patent Office

Office européen des brevets

(11) Publication number: **0 138 784**
A2

# EUROPEAN PATENT APPLICATION

(21) Application number: **84850245.6**

(22) Date of filing: **20.08.84**

(51) Int. Cl.⁴: **C 08 B 37/00, A 61 K 31/715**

(30) Priority: **08.09.83 SE 8304821**

(43) Date of publication of application: **24.04.85**
**Bulletin 85/17**

(84) Designated Contracting States: **AT BE CH DE FR GB IT LI NL SE**

(71) Applicant: **BioCarb AB, Box 739, S-220 07 Lund (SE)**

(72) Inventor: **Svensson, Sigfrid, Södra Ljungvägen 10, S-244 02 Furulund (SE)**
Inventor: **Lindberg, Alf, Johan Banérs väg 46, S-182 75 Stocksund (SE)**
Inventor: **Lundblad, Arne, Ängsvägen 2A, S-752 45, Uppsala (SE)**

(74) Representative: **Burman, Tore et al, Bergling & Sundbergh AB P.O. Box 7645, S-103 94 Stockholm (SE)**

(54) Arabinogalactans, their preparation and compositions containing same.

(57) Arabinogalactans recoverd from trees of the genus Cocos for use as a therapeutic, prophylactic or diagnostic agent in connection with the presence of microorganisms; a process for the manufacture of the arabinogalactans; and a composition containing same.

EP 0 138 784 A2

19922 TB/LF
26.7.1984

0138784

TITLE OF INVENTION
Arabinogalactans, their preparation and compositions
containing same.

The present invention relates to arabinogalactans
which are useful as therapeutic, prophylactic or diagnostic
agents in connection with the presence of microorganisms.
The invention also includes processes for manufacturing the
arabinogalactans and a composition containing same.

The present invention thus relates to compositions
and arabinogalactans useful for therapeutic treatment of infections caused by patogenic microorganisms, such as bacteria,
particularly intestinal bacteria, such as Gram-negative types.
The invention is particularly applicable to bacterias of the
genus Enterobacteriaceae, such as Escherichia coli bacteria,
particularly K88+. The compositions of the invention are useful also for prophylactic and diagnostic procedures. The invention also includes a process for therapeutic treatment of
mammals including man. Although the invention is not limited
in such a way it will in the following be illustrated essentially in connection with the bacterium E.coli.

Bacterial infection courses are often initiated by the
fact that the bacterium has the ability to adhere to epithelial tissue. This adhereing capacity is specific in that different bacteria adhere preferentially to different types of
tissues. Thus, studies have shown that the bacterium E.coli
possessing fimbriae (pili) designated K-antigene K88 adheres
well to intestinal epithelium of piglets (ref. 1). It has also been shown that the K88 fimbriae are responsible to the ability of E.coli K88+ to agglutinate guinea-pig erythrocytes
(ref. 2). The mutant lacking K88-fimbriae lacks ability to adhere to intestinal epithelium with the result that said E.coli
bacteria cannot colonize the intestinals of pigs (ref. 3). It
has, moreover, been shown that the K88-fimbriae recognize some
structure on the "brush border" membranes of the intestinal epithelium. Thus, it has been shown that certain pigs lack this structure in the intestinal epithelium and that this fact results in said pigs being resistant to intestinal infections caused by E.coli K88+.

The present invention accordingly provides for arabinogalactans from trees of the genus Cocos or compositions containing such arabinogalactans having the ability of replacing the normal receptor in vivo in relation to patogenic microorganisms capable of providing infections in man and animals.

Another object of the invention is to provide such composition or substance which in addition to the therapeutic use thereof also can be used diagnostically.

The expression "microorganism" used in the present disclosure is intended to include bacteria, viruses, animal cells and plant cells. The invention is particularly directed to the receptor structure for K88-fimbriated E.coli but is not limited to this type of bacteria.

The arabinogalactans involved in the present invention may thus be used therapeutically, prophylactically or diagnostically. The arabinogalactans according to the invention are preferably recovered from trees of the species Cocos nucifera, i.e. trees of the palmtype. It is mainly the cocoa-nuts of the trees which contain the compounds of interest, i.e. the milk of the cocoa-nut or its meat, for example as available in dried form under the name copra.

As previously indicated the invention is particularly applicable to diarrhoea-generating microorganisms, for example E.coli K88+ or closely related bacteria.

The invention also provides for a process for recovering such arabinogalactans or solvolysates thereof from cocoa-nut meat, dried such meat, i.e. copra, or defatted cocoa-nut meat. Such recovery is suitably performed by extraction while using a hydrofilic extraction means, preferable a water-based extraction agent. In such extraction there will be obtained a hydrofilic base from which the desirable arabinogalactans can be recovered in a suitable manner, for example by freezedrying, evaporation or the like. It is preferred before recovering the arabinogalactans from the hydrofilic phase to subject same to separation of low molecular inactive materials, which may be present for example in the form of salts, degradation products, other biproducts or the like.

The active arabinogalactans according to the present invention may in a conventional manner be formulated for use in human or veterinary medicine for therapeutic, prophylactic os diagnostic purposes. The composition or the pharmaceutical preparation may contain the active arabinogalactans in combination with a pharmaceutically acceptable carrier which may be solid, semi-solid or liquid depending on the manner of administration and further factual circumstances. The active substances may also be used as such without addition of carrier materials. The compositions are prepared in full comformity with conventional pharmaceutical practice.

Suitable forms of the compositions of this invention include tablets, capsules, syrups, suspensions, solutions, concentrates, reconstitutable powders in sterile forms suitable for injection or infusion. Such compositions may contain conventional pharmaceutically acceptable materials, such as diluents, binders, colouring agents, flavouring agents, preservatives, desintegrants and the like in accordance with conventional pharmaceutical practice in the manner well understood by those skilled in the art of formulating antibiotics.

According to a preferred aspect of the present invention the present compositions are used for therapeutic or prophylactic purposes to treat or prevent diarrhoea in pigs, particularly piglets. For therapeutic purposes the active arabinogalactans are suitably dissolved in the drinking water of the animal, and in particularly severe cases it can be administered by oral gavage.

For prophylactic purposes the active substance may be added directly to the animal´s diet, either _per_ _se_ or as a concentrate in aqueous solution or other suitable formulation, for example together with a diluent to facilitate dosage.

Suitable quantities can be determined on a case to case basis and may be of the order of grams per day, for example 0.1-50 grams per day.

The invention will in the following be further described by non-limiting examples.

<u>Inhibition of heamagglutination of guinea-pig erythrocytes with E.coli K88+ or k88-fimbriae (pili) by addition of material according to the invention containing arabinogalactans.</u>

In the tests bacteria of the type <u>E.coli</u> O 149 K88+ were used. The bacteria were cultivated on CFA-medium at +37$^o$C for 18 hours. The bacterial growth wass slurried in PBS, pH 7.2 to a density of $1 \times 10^{10}$ bacteria/ml. K88-fimbriae were obtained in the following manner.

The bacterial strain was cultivated on Roux flasks with CFA-agar. After incubation at +37$^o$ for 24 hours the bacteria were harvested by adding 10 ml PBS/flask and sterile glass balls. The flasks were shaken and the bacterial suspension removed. The bacteria were pelletized by centrifugation (3000 x g, +4$^o$, 30 min.). The supernatant was removed by suction. The bacteria were resuspended in 10 ml PBS and the suspension was treated in a Waring blendor 2 x 20 seconds. The suspension was centrifuged (5000 x g, +4$^o$, 30 min.) to pelletize the bacteria. The supernatant containing fimbriae was removed by suction. The bacterial pellet is resuspended, treated in a Waring blendor and after centrifugation the supernatant is again recovered. The two supernatants are pooled and ammonium sulphate is added to a concentration of 30%. After stirring the supernatants are allowed to stand at +4$^o$C for 18 hours, centrifugation then taking place at 5000 x g. The supernatant was separated and the precipitation was dialyzed against PBS. The solubilized fimbriae fraction was precipitated with ammonium sulphate once more and dialyzed.

Haemagglutination is performed using erythrocytes from guinea-pigs (GRBC) suspended to a concentration of 3% v/w in PBS. The agglutination tests were carried out so that 50 $\mu$l GRBC were mixed with 2-step dilution of antigen 50 $\mu$l bacterial suspension or 50 $\mu$l fimbriae suspension and 50 $\mu$l PBS in microtitre plates. The plates were sealed with Para-

5

0138784

film and incubated at +44°C for 4 hours and were then recorded. The last well wherein agglutination could be read by the eye is considered as 1 haemagglutinating unit ( 1 HU).

In the inhibition experiments there were added instead of PBS aqueous solutions of arabinogalactans the inhibiting capacity of which was studied. These solutions were tested by 2-step dilution. In the tests there were used concentrations of bacteria and fimbriae which by 3% GRBC results in 2-4 HU. The results are presented in Table 1.

Example 1.

Preparation of active arabinogalactan from cocoa-nut milk.

Cocoa-nut milk (22 litres) were concentrated to 2 liters by evaporation, and after centrifugation flocculated fat was removed by filtration. The solution was dialyzed and the high molecular fraction, molecular weight > about 5000, (30g) were passed through a column packed with Sephadex G15. The "Void"-fraction from the column was collected (25g). Analysis of the fraction showed that it contained a polysaccharide containing arabinose and galactose units.

The fraction obtained was hydrofilized and the residue was dissolved in water to form an aqueous solution subjected to 2-step serial dilution for later use in inhibition of haemagglutination. The results of the inhibition experiments are given in Table 1 below.

Example 2.

Preparation of active arabinogalactans from so called expellor.

14.9 kg of expellor, i.e. the residue of the meat of the cocoa-nut after removal of the fat, are comminuted by grinding in a mill, 100 litres of water being then added. The extraction is performed at 100°C for a period of time of 4 hours and results after precipitation with 70% ethanol-water about 1 kg of solid material.

This material is then dissolved in water and insoluable material is removed by centrifugation. 2-step serial dilution is then performed and the solutions obtained are tested in inhibition experiments. The results of the inhibition experiments are given in Table 1 below.

Example 3.

Example 2 is repeated while using so called copra which is constituted by dried meat from cocoa-nut. After separation of fat in connection with the process there is obtained an aqueous solution which is subjected to 2-step serial dilution. The results of the inhibition experiments are given in Table 1 below.

Table 1. Inhibition of haemagglutination between GRBC and K88-fimbriated bacteria or K88-fimbriae.

| Inhibitor | Minimum inhibitory concentration $\mu$g/ml |
|---|---|
| Example 1 | 200 |
| Example 2 | 500 |
| Example 3 | 400 |

Clinical tests

For the purpose of studying the inhibitory effect of cocoa-nut milkglycosides on diarrhoea in piglets caused by E.coli K88 clinical testing was performed. The animals used were 6 piglets released by sectio caesarea on day 1. These piglets had accordingly up to the clinical testing never been confronted with any environmental hazards including foreign microorganisms. The piglets were divided up into 2 groups, one group being designated I and the other group being designated II. The first group I was challenged with Coli 0149 (LT$^+$ ST$^+$ K88$^+$) in combination with an aqueous solution of the cocoa-nut milk arabinogalactan prepared in accordance with Example 1 above. The other group II was challenged with the same E.coli only. Group I = 4 piglets and group II = 2 piglets.

On day 2 the following schedule was exercised.

| Time day 2 | Group I | Group II |
|---|---|---|
| 10.20 | 0.5 g glycoside,6 ml aqueous solution,orally | 6 ml water, orally |
| 11.20 | $1.4 \times 10^7$ bacteria,orally | $1.4 \times 10^7$ bacteria,orally |

| Time day 2 | Group I | Group II |
|---|---|---|
| 11:30 | 0.5g glycoside,6 ml aqueous solution,orally | 6 ml water, orally |
| 13.00 | 70 ml of milk | 70 ml of milk |
| 14.30 | 0.5 g glycoside,6ml aqueous solution,orally | 6 ml water, orally |
| 15.00 | 70 ml of milk | 70 ml of milk |
| 19.30 | 0.5 g glycoside,6ml aqueous solution,orally | 6 ml water, orally |
| 20.00 | 70 ml of milk | 70 ml of milk |

At 23.00 hours, day 2, clinical controll of the piglets was made. No irregular observations were encountered except that 10 hours after the challenge with bacteria two out of the four piglets of Group I and both piglets of Group II showed slight diarrhoea.

On day 3 at 05.00 hours renewed clinical controll was made. All 4 piglets of Group I showed good health, whereas both piglets of Group II were moribund and could not stand on their legs. At 06.00 hours 2 piglets out of Group I and both piglets out of Group II were sacrified for autopsy. At 06.30 hours, day 3, the two remaining piglets of Group I were given orally 0.5 g of glycoside in the form of a 6 ml aqueous solution. At 09.00 hours the same day the two piglets were again given orally the same amount of glycoside solution together with 70 ml of milk. At this time the piglets were clinically healthy. At 11.00 hours they were still clinically healthy but both piglets showed fluid diarrhoea. At 14.00 hours, day 3, the two piglets became affected by diarrhoea and were sacrified.

Autopsy

The two piglets of Group I sacrified 06.00, day 3, showed healthy intestinals, and one of the piglets suffered some liquid release in jejunum, ileum and colon.

The two piglets from Group II showed strong interior haemorrhage and liquid release in jejunum and ileum.

It can be seen from the results of the above clinical tests that the glycoside used in accordance with the invention had a significant influence on the reaction of the piglets due to the heavy bacterial challenge. It should be noted in this connection that the piglets subject to testing were obtained by sectio caesarea and thus totally unaffected by all environmental influence whatsoever. In this virginal state the piglets are, of course, from an immunological point of view quite hypersensitive and the bacterial treatment thus highly drastic. The effect of administering the glycoside must be seen against this factual background.

0138784

References:

Ref. 1   Jones, G.W. and Rutter, J.M.
         Infection and Immunity 6 (1972) 918-927.

Ref. 2   Jones, G.W. and Rutter, J.M.
         J.Gen. Microbiol. 84 (1974) 135-44.

Ref. 3   Sellwood, R., Gibbons, R.A., Jones, G.W.
         and Rutter, J.M.
         J. Med.Microbiol. 8 (1975) 405-411.

0138784

CLAIMS

1.    Arabinogalactans recovered from trees of the genus Cocos for use as a therapeutic, prophylactic or diagnostic agent in connection with the presence of microorganisms.

2.    Arabinogalactans according to claim 1 recovered from the species Cocos Nucifera.

3.    Arabinogalactans according to claim 1 or 2 recovered from cocoa-nut milk.

4.    Arabinogalactans according to claim 1 or 2 recovered from cocoa-nut meat, particularly dried meat (copra).

5.    Arabinogalactans according to any preceding claim for use in connection with diarrhoe-generating microorganisms, particularly such organisms the receptors of which are or correspond to those of E.coli K88+or closely related bacteria.

6.    A process for recovering arabinogalactans or solvolysates thereof from cocoa-nut meat, dried such meat (copra) or defatted cocoa-nut meat, characterized thereby that the recovery takes place by means of extraction while using a hydrofilic extraction agent.

7.    A process according to claim 6, characterized thereby that the extraction agent is aqueous.

8.    A process according to claim 7, characterized thereby that the hydrofilic phase obtained in the extraction is subjected to separation of low molecular inactive materials, for example salts, degradation products, biproducts or the like and that then the desired arabinogalactans are recovered.

9.    Therapeutically, prophylactically or diagnostically useful composition comprising arabinogalactans according to any of claims 1 - 5 and a carrier acceptable for the purpose.